# EUROPÄISCHE PATENTSCHRIFT

(11) **EP 2 299 982 B1**
(45) Veröffentlichungstag und Bekanntmachung des Hinweises auf die Patenterteilung: **04.04.2018**
(21) Anmeldenummer: 09765502.1
(22) Anmeldetag: 25.05.2009
(51) Int. Cl.: A61K 9/20

(54) **DIREKT VERPRESSBARE UND SCHNELL ZERFALLENDE TABLETTENMATIRX**
DIRECTLY INJECTION MOLDABLE AND RAPIDLY DISINTEGRATING TABLET MATRIX
MATRICE POUR COMPRIMÉ DIRECTEMENT COMPRESSIBLE ET À DÉSINTÉGRATION RAPIDE

(30) Priorität: 20.06.2008 EP 08011238; 11.08.2008 EP 08014287
(43) Veröffentlichungstag der Anmeldung: 30.03.2011
(73) Patentinhaber: Merck Patent GmbH, 64293 Darmstadt (DE)
(72) Erfinder: KLEINWAECHTER, Daniela, 64289 Darmstadt (DE); MODDELMOG, Guenter, 64354 Reinheim (DE); OGNIBENE, Roberto, 64297 Darmstadt (DE)
(86) Internationale Anmeldenummer: PCT/EP2009/003677
(87) Internationale Veröffentlichungsnummer: WO 2009/152922

(56) Entgegenhaltungen:
- WO-A-2006/097946
- WO-A-2007/076874
- WO-A1-2007/060402
- DE-A1- 4 439 858
- US-A1- 2004 162 333

## Beschreibung

Gegenstand der vorliegenden Erfindung ist eine neue direkt verpressbare Matrix zur Herstellung von in Gegenwart von Feuchtigkeit schnell zerfallenden Tabletten.

### Stand der Technik

Zur schnellen Wirkstofffreisetzung aus festen oralen Arzneiformulierungen, insbesondere aus Tabletten, bedarf es einer sehr schnell zerfallenden, angenehm schmeckenden und einfach zu verarbeitender Tablettenmatrix. Gleichzeitig muss diese Matrix durch Kompression genügend harte Tabletten mit geringem Abrieb liefern, um sie nach ihrer Herstellung für die Weiterverarbeitung, wie z.B. zur Abpackung und für den Patienten gut handhabbar zu machen.

Mannit bietet sich als Grundlage für solch eine Matrix an, da es nach geeigneter Vorbehandlung gut komprimierbar ist, eine gute Lagerstabilität aufweist, mit fast allen Wirkstoffen kompatibel ist und gleichzeitig angenehm süß schmeckt.

Die derzeit auf dem Markt befindlichen Mannitzubereitungen für diesen Einsatzzweck sind jedoch entweder sehr komplex aufgebaut und/oder in ihren galenischen Eigenschaften unzureichend.

In WO 2006/097946 A (ACTAVIS GROUP HF [US]) wird eine Formulierung zur Herstellung von Topiramat enthaltenden Tabletten beschrieben, welche 25 bis 70 Gew.-% sprühgetrocknetes, granuliertes Mannit und Topiramat in einer Menge von etwa 5 bis 35 Gew.-% enthalten. Diese Zusammensetzungen können Tablettensprengmittel, wie vernetztes Polyvinylpyrrolidon, Maisstärke, modifizierte stärke, vernetzte Natriumcarboxycellulose, Natriumstärkeglykolat und Miaschungen davon enthalten. Jedoch sind in dieser Schrift keine weiteren Informationen und Daten zu den erhaltenen Formulierungen enthalten.

In WO 2007/060402 A1 (ASTRAZENECA [UK]) wiederum wird eine pharmazeutische Zusammensetzung beschrieben, die den schwer tablettierbaren, plastischen Wirkstoff AZD2171 enthält, und aus der Tablettenkerne hergestellt werden, die beschichtet werden können. Die in dieser Schrift beschriebenen Zusammensetzungen enthalten schwer tablettierbaren Wirkstoff, einen offenporigen plastischen Füllstoff, ausgenommen Lactose, einen spröden Füllstoff mit niedriger Oberflächenacidität, einen zweiten plastischen Füllstoff und als letztes ein Tablettensprengmittel. Als Tablettensprengmittel wird vorzugsweise Natriumstärkeglykolat eingesetzt.

### Aufgabenstellung

Zwei gegenläufige Anforderungen an solch eine Tablettenmatrix, und zwar hohe Tablettenhärten bei gleichzeitig sehr schnellem Zerfall und Freisetzung der Wirkstoffe, sind in einer solchen geeigneten Zubereitung zu vereinigen. Weiterhin sollte sich die Matrix in der industriellen Tablettenherstellung sehr einfach verarbeiten lassen, so dass sie sich mit den übrigen Tablettenkomponenten ohne Granulierungsschritte direkt verpressen lässt.

### Lösung der Aufgabe

Durch Versuche wurde nun gefunden, dass sich durch die Bereitstellung einer Co-Mischung eines speziellen Mannitpulvers mit quervernetzter Natriumcarboxymethylcellulose eine Tablettenmatrix mit einer speziellen Kornstruktur und großer BET-Oberfläche zur Herstellung schnell zerfallender Tabletten in einem Direkttablettierungsprozess erhalten werden.

Gegenstand der vorliegenden Erfindung ist somit eine Co-Mischung zur Herstellung schnell zerfallender Tabletten in einem Direkttablettierungsprozess, bestehend aus 90 bis 98 Gewichtsteilen, besonders bevorzugt 95 Gewichtsteilen, eines gesprühten Mannits und 10 bis 2 Gewichststeilen, besonders bevorzugt 5 Gewichtsteilen, einer quervernetzten Natriumcarboxymethylcellulose, welche dadurch charakterisiert ist, dass die Co-Mischung eine BET-Oberfläche größer als 1,5 m²/g aufweist bei einem mittleren Partikeldurchmesser (Laser) im Bereich zwischen 60 und 200 µm, zu Tabletten verpressbar ist und nach Kompression bei einer Preßkraft von 20 kN Tabletten mit Härten >250N, einer Friabilität ≤0,14% und einer Zerfallszeit ≤70 Sekunden erhalten werden. Als besonders vorteilhaft hat es sich erwiesen, wenn Mischungen verwendet werden, deren BET-Oberfläche im Bereich von 1,9 bis 4,0 m²/g liegt, insbesondere im Bereich von 1,9 bis 2,6 m²/g. Als ganz besonders vorteilhaft hat es sich erwiesen, wenn entsprechende Mischungen zur Herstellung von Tabletten eingesetzt werden, die gleichzeitig eine Schüttdichte im Bereich von 0,45 bis 0,60 g/ml, einer Stampfdichte im Bereich von 0,60 bis 0,75 g/ml, sowie einen Fließneigungswinkel im Bereich von 30 bis 38° aufweisen.

In solchen erfindungsgemäßen Co-Mischungen können die Partikel mittlere Partikeldurchmesser (Laserbestimmung) im Bereich zwischen 60 und 200 µm, bevorzugt im Bereich von 64 - 114 µm aufweisen. Vorzugsweise besitzt die einzusetzende Mischung einen Wassergehalt <1 Gew.-%.

Co-Mischungen, welche diese Eigenschaften aufweisen, sind in einfacher Weise zu Tabletten verpressbar.

Insbesondere sind die erfindungsgemäßen Co-Mischungen geeignet zur Verwendung als Trägermaterial für Wirkstoff- und/oder aromastoffhaltige Tablettenformulierungen, welche die genannten vorteilhaften Eigenschaften aufweisen, sowie aus diesem Trägermaterial hergestellte Tabletten oder andere pharmazeutische Formulierungen mit oder ohne Wirkstoff.

Aufgrund ihrer vorteilhaften Eigenschaften sind die erfindungsgemäßen Trägermaterialien besonders zur Verwendung für die Herstellung von Tablettenformulierungen geeignet, welche Wirkstoffe und Aromastoffe enthalten. Diese können insbesondere sein:
- Substanzen aus dem Bereich Nahrungsergänzung wie z.B. Vitamine, Mineralstoffe, Spurenelemente, funktionelle Lebensmittelbestandteile z.B. Pflanzenbestandteile und Pflanzenextrakte
- Substanzen aus dem Bereich synthetische und natürliche Farbstoffe, natürliche und naturidentische Aromastoffe und andere geschmacksgebende Substanzen wie z.B. Süßstoffe (Aspartam, Sachcharin, Acesulfam K, Neohesperidin DC, Sucralose; Thaumatin, Steviosid), Fruchtaromen, Pfefferminzaromen oder Menthol, Fuchtsäuren, wie Citronensäure und Weinsäure, geschmacksgebende Pflanzenauszüge, etc.
- Substanzen mit pharmakologischer Wirkung wie z.B. Antacida, Antiinfektiva, welche auch zur lokalen Wirkung im Mund- und Rachenraum eingesetzt werden,
- Analgetika einschl. Opioide, Antiallergika, Antiemetika, Antidiarrhoika, etc.

### Detaillierte Beschreibung der Erfindung

Durch Kombination (Mischung) eines gesprühten Mannits (Parteck M, Merck KGaA) mit einem sogenannten Supersprengmittel z.B. Na-CMC quervernetzt (Ac-Di-Sol, FMC BioPolymer) läßt sich eine direkt verpressbare Tablettenmatrix erhalten, die über einen sehr weiten Härtebereicherreich konstant niedrige Zerfallseigenschaften zeigt. Diese sehr niedrigen Zerfallszeiten sind über einen deutlich weiteren Härtebereich aufrecht zu erhalten als dies mit co-gesprühten Mannit-Sprengmittel-Kombinationen (z.B. Ludiflash; BASF) oder durch Abmischungen mit anderen im Handel erhältlichen direkt verpressbaren Mannittypen (DC-Mannit) mit quervemetztem Na-CMC der Fall ist. Überraschend ist zudem, dass durch den Mischungsvorgang trotz der erheblichen mechanischen Belastung die Direktverpressungseigenschaften (DC-Eigenschaften) des Mannits nicht leiden.

Mit dem erhaltenen Material kann der Weiterverarbeiter daher durch einfaches Abmischen mit seinen Rezepturbestandteilen und nachfolgende Direktverpessung auf einfachem Wege sehr schnell zerfallende Tabletten, z.B. ODT (orally dispersible tablet), erhalten. Dieser sehr schnelle Zerfall ist eine wesentliche Voraussetzung für eine schnelle Wirkstofffreisetzung, verbunden mit einer schnellen Resorption des APIs, beispielsweise bereits im Mund und Rachenraum.

Eine Abmischung (z.B. im Schnecken-Kegel-Mischer) einer Kombination, bestehend aus 90-98 Gew.-Teilen Parteck M (bevorzugt Parteck M200) mit 10-2 Gew.-Teilen eines Supersprengmittels, bevorzugt mit quervernetztem Na-CMC z.B. Ac-Di-Sol erfüllt in hervorragender Weise diese Anforderungen. Die besten Ergebnisse hinsichtlich des Härte/Zerfallszeit-Verhältnisses werden mit einer Kombination, bestehend aus 95% Parteck M200 und 5% Ac-Di-Sol Typ SD-711; FMC BioPolymer, erhalten

Die Herstellung des erfindungsgemäß einsetzbaren pulverförmigen Mannits kann nach einem aus der Patentschrift EP 0 896 528 B1 bekannten Verfahren erfolgen. Bevorzugt erfolgt die Herstellung durch ein entsprechendes Sprühtrocknungsverfahren. Sie kann aber auch durch Wirbelschichtgranulation erfolgen. Die Durchführung des Verfahrens erfolgt an sich, indem
a) eine wässrige Mannitlösung hergestellt wird, die evtl. ein weiteres Polyol in einer geringen Menge enthalten kann, gegebenenfalls in einer Menge von bis zu 2 Gew.-% bezogen auf die Gesamtmenge an Mannit und weiterem Polyol, und
b1) Versprühen der erhaltenen Lösung in einem Luftstrom mit einer Temperatur zwischen 120 und 300 °C, wobei das Wasser der Lösung verdampft wird, oder
b2) Verwirbeln der erhaltenen Lösung in einem Luftstrom mit einer Temperatur zwischen 40 und 150 °C, wobei das Wasser der Lösung verdampft wird.

Die Versprühung wird durch Zerstäuben mittels Düsen, vorzugsweise mittels eines Zentrifugalzerstäubers, in einen auf eine Temperatur von 120 bis 300° C, vorzugsweise 140 bis 190 ° C erwärmten, trockenen, zentrifugal eingeblasenen Luftstrom durchgeführt. Die Menge der zugeführten Polyollösung und der eingeblasenen Heißluft wird so aufeinander abgestimmt, dass das Polyol bis auf einen Wassergehalt von etwa 0,3 bis etwa 1 Gew.-% getrocknet wird. Auf jeden Fall sollte der Wassergehalt unterhalb 1 Gew.-% liegen.

Die Polyolagglomerate, die durch Entwässerung der Polyollösungströpfchen erhalten werden, werden bei der Sprühtrocknung auf eine Temperatur von etwa 50 bis etwa 70 ° C erwärmt, während sich die eingeblasene Luft auf ungefähr die gleiche Temperatur abkühlt. Die so hergestellte Mannitqualität wird in Behältern gesammelt und ist nach dem Abkühlen direkt zur Herstellung von Tabletten bzw. Komprimaten geeignet.

Es ist aber auch möglich, die Herstellung des erfindungsgemäß einsetzbaren Mannits in einer Anlage mit Wirbelbett zur Nachtrocknung des sprühgetrockneten Materials gegebenenfalls mit Pulverrückführung in einer kontinuierlicher Fahrweise durchzuführen. Entsprechende Anlagen sind dem Fachmann bekannt, wie z. B. aus den Patentschriften EP 1 453 781 B1 und EP 1 319 644 B1 bzw. aus WO 00/76650 A1. Solche Anlagen erlauben es dem Fachmann durch gezielte Einstellung des Pulverbettes, des Gasstroms, der Temperaturführung und gegebenenfalls zusätzliches Aufsprühen von zusätzlicher Ausgangslösung, sowie einer gesteuerten Produktrückführung von feinem Material, die gewünschte Korngröße des Granulats einzustellen. Einfachere Anlagen finden sich in den Patentschriften EP 0738 252 B1 oder EP 0904059 B1.

Das so erhaltene Mannit weist neben einer filamentösen Mikrostruktur eine sehr große Oberfläche auf. Das gewünschte Korngrößenspektrum der in dem hergestellten Pulver enthaltenen Partikel lässt sich gezielt durch die Wahl der Betriebsparameter der Sprühtrocknungsanlage bzw. Wirbelschichtgranulationsanlage einstellen. Es ist aber auch möglich, durch Sieben Über- und Unterkorn abzutrennen und so die mittleren Partikeldurchmesser des Mannitpulvers im Bereich von 60 bis 200 µm, bevorzugt im Bereich von 64 bis 114 µm einzustellen. Entsprechende Methoden zum Sieben unter milden Bedingungen sind dem Fachmann bekannt. Im Handel sind entsprechende Produkte unter dem Markennamen Parteck M, z. B. Parteck M 200 erhältlich.

Wie oben bereits gesagt, wurde durch Versuche gefunden, dass eine Co-Mischung, bestehend aus einem Mannit der beschriebenen Qualität und einem sogenannten Supersprengmittel in einem bestimmten Mischungsverhältnis bezogen auf das Gewicht, ein Trägermaterial für die Herstellung von Tabletten zubereitet werden kann, dass bereits beim Verpressen mit relativ geringer Preßkraft Tabletten mit vergleichsweise hohen Tablettenhärten liefert, welche jedoch in Gegenwart von Feuchtigkeit in relativ kurzer Zeit zerfallen.

Als geeignetes Supersprengmittel hat sich in diesem Zusammenhang im Handel erhältliche quervernetzte Na-CMC erwiesen, welche im Handel unter verschiedenen Handelsnamen erhältlich ist und ebenfalls geringe mittlere Partikeldurchmesser bei gleichzeitig ausreichend großer Oberfläche aufweist. Bevorzugt wird quervernetzte Na-CMC mit mittlerem Partikeldurchmesser im Bereich von 10 bis 100 µm, bevorzugt im Bereich von 20 bis 50 µm, eingesetzt.

Durch Vergleichsversuche wurde gefunden, dass die erfindungsgemäßen Produkte, bestehend aus einer Mischung von 90-98 Teilen Parteck M mit 10 bis 2 Teilen eines Supersprengmittels, bevorzugt mit quervernetztem Na-CMC, z.B. Ac-Di-Sol, im Vergleich zu den bekannten gesprühten Mannittypen ohne Zusatz von quervernetzter Na-CMC als Trägermaterial, insbesondere im Bereich höherer Preßkräfte, verbesserte Tablettenhärten zeigen, d.h. dass sie vor allem keine Neigung zum "Deckeln" zeigen. Zudem sind die Zerfallszeiten der erfindungsgemäßen Produkte deutlich geringer als die unter Verwendung von gesprühten Mannittypen ohne Zusatz von quervernetzter Na-CMC als Trägermaterial erhaltenen Tabletten.

Ein co-gesprühtes Produkt, enthaltend 95 Teile Mannit und 5 Teile quervernetzter Na-CMC weist zwar, verglichen mit den erfindungsgemäßen Produkten, ähnliche Tablettenhärten (und zeigt auch keine "Deckelneigung") auf, besitzt dabei jedoch deutlich höhere Zerfallszeiten. Dieses Vergleichmaterial wird nach dem beschriebenen Verfahren hergestellt, indem abweichend die quervernetzte Na-CMC in der zu sprühenden Mannitlösung entsprechend dem Verhältnis Mannit zu quervernetztes Na-CMC 95 : 5 (als Trockensubstanzen) verteilt und unter laufender Agitation zusammen unter den beschriebenen Bedingungen durch Sprühtrocknung oder Wirbelschichtgranulation co-gesprüht wird."

Es wurde auch gefunden, dass die Zerfallszeiten von Tabletten aus erfindungsgemäßen Co-Mischungen im Vergleich zu Tabletten, welche erhalten werden aus handelsüblichen Produkten, über einen weiteren Härtebereich hinaus niedrig sind. Daher ist das erfindungsgemäße Material besonders gut zur Formulierung von pharmazeutischen Zusammensetzungen in Direkttablettierungsprozessen geeignet, und zwar insbesondere auch für die Herstellung von in der Mundhöhle schnell Wirkstoff-freisetzende Tabletten.

Weiterhin lassen sich durch Verwendung der erfindungsgemäßen Co-Mischung über einen größeren Tablettenhärtebereich verhältnismäßig schnell zerfallende Tabletten erhalten im Vergleich zu Tabletten, hergestellt mit dem für ODT Formulierungen im Handel beworbenen, co-gesprühten Mannit/Crospovidone/Polyvinylacetat/Povidone (Ludiflash). Außerdem lassen sich, wie bereits erwähnt, bei Anwendung vergleichbarer Preßkräfte mit den erfindungsgemäßen Mischungen höhere Tablettenhärten erzielen. Dieses ist für den Galeniker für die Formulierung schlecht komprimierbarer Wirkstoffe von besonderem Vorteil und ermöglicht ihm eine leichtere Durchführung der Tablettierung.

Aber auch Pearlitol 200 SD, welches ebenfalls ein gesprühtes Mannit ist, zeigt nach Abmischung mit AcDiSol im Vergleich zu der erfindungsgemäßen Co-Mischung ein schlechteres Tablettierverhalten, ebenso wie andere direkt verpressbare Mannit-Typen auf kristalliner bzw. granulierter Basis, die sich durch noch schlechtere Bindungseigenschaften, insbesondere hohe Friabilitäten, auszeichnen.

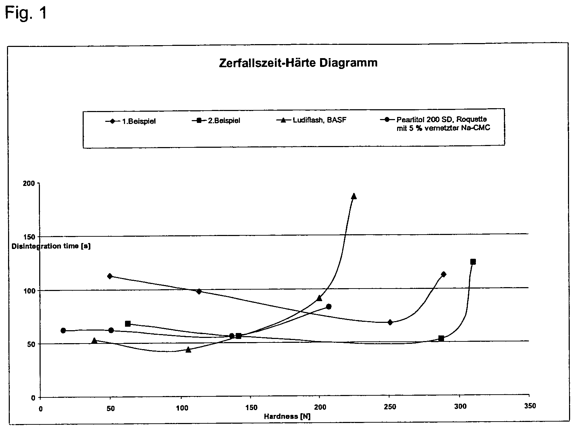

Die Ergebnisse der durchgeführten, erfindungsgemäßen Tablettierversuche und der Vergleichsbeispiele sind in Fig. 1 graphisch dargestellt, wobei die Zerfallszeit der hergestellten Tabletten gegen die erhaltenen Tablettenhärten aufgetragen sind.

In vorteilhafter Weise können bei Verwendung der erfindungsgemäßen Co-Mischungen als Matrix zur Tablettenherstellung bei vergleichbaren Preßkräften härtere Tabletten erhalten werden im Vergleich zur Verwendung von Trägermaterialien, welche dem Fachmann aus der Literatur und dem Handel bekannt sind.

Weiterhin wurde gefunden, dass durch Verwendung des erfindungsgemäßen Materials bereits sehr gute Bindungseigenschaften erhalten werden, wenn zur möglichsten Schonung der Tablettierwerkzeuge und Tablettiermaschinen in einem bevorzugten niedrigen Preßkraftbereich von bis 20 kN tablettiert wird. Außerdem zeigen die erhaltenen Tabletten eine sehr gute mechanische Stabilität, gemessen als Friabilität mit deutlich <1%, auf, wodurch eine sichere Handhabung der Preßlinge gewährleistet ist.

Die vorliegende Beschreibung ermöglicht es dem Fachmann, die Erfindung umfassend anzuwenden. Auch ohne weitere Ausführungen wird daher davon ausgegangen, dass ein Fachmann die obige Beschreibung im weitesten Umfang nutzen kann.

Bei etwaigen Unklarheiten versteht es sich von selbst, die zitierten Veröffentlichungen und Patentliteratur heranzuziehen. Dementsprechend gelten diese Dokumente als Teil der Offenbarung der vorliegenden Beschreibung.

Zum besseren Verständnis und zur Verdeutlichung der Erfindung werden im folgenden zwei Beispiele gegeben, die im Rahmen des Schutzbereichs der vorliegenden Erfindung liegen. Diese Beispiele dienen auch zur Veranschaulichung möglicher Varianten. Aufgrund der allgemeinen Gültigkeit des beschriebenen Erfindungsprinzips sind die Beispiele jedoch nicht geeignet, den Schutzbereich der vorliegenden Anmeldung nur auf diese zu reduzieren.

Weiterhin versteht es sich für den Fachmann von selbst, dass sich sowohl in den gegebenen Beispielen als auch in der übrigen Beschreibung die in den Zusammensetzungen enthaltenen Komponentenmengen in der Summe immer nur zu 100 Gew. bzw. mol-% bezogen auf die Gesamtzusammensetzung aufaddieren und nicht darüber hinausgehen können, auch wenn sich aus den angegebenen Prozentbereichen höhere Werte ergeben könnten. Sofern nichts anderes angegeben ist, gelten daher %-Angaben als Gew.- oder mol-%, mit Ausnahme von Verhältnissen, die in Volumenangaben wiedergegeben sind.

Die in den Beispielen und der Beschreibung sowie in den Ansprüchen gegebenen Temperaturen gelten immer in °C.

### Beispiele

Zur Charakterisierung des Materials werden folgende analytischen Verfahren angewandt:
- Schüttdichte: gemäß DIN EN ISO 60
- Stampfdichte: gemäß DIN EN ISO 787-11: 1995
- Schüttwinkel: gemäß DIN ISO 4324
- Bestimmung der Partikelgrößeverteilung: Laserstreuung mit Trockendispergierung mit Mastersizer 2000 Version 5.10 G, Serial Number: 34403-97 von Malvern Instruments Ltd; Dispergiereinheit Scirocco 2000 (A), Gegendruck 1 bar; Auswertemodell: Universal; Fraunhofer; Ausführung gemäß technischem Handbuch und Spezifikationen des Herstellers
- Tablettierungsprüfung:
   495 g des auf seine Tablettierungseigenschaften zu prüfenden Materials werden mit 5g Parteck LUB MST (Magnesiumstearat pflanzlich) EMPROVE exp PhEur, BP, JP, NF, FCC Art.Nr. 1.00663 (MERCK KGaA, Deutschland) versetzt, das Magnesiumstearat wird zuvor über ein 250µm Sieb abgelegt, und 5 Minuten in einem verschlossenen Edelstahlbehälter [Fassungsvolumen: ca. 2 I, Höhe: ca.19,5 cm, Durchmesser: ca.12 cm, Außenmaße] auf einem Labor-Taumelmischer [Turbula, Fa. Willy A. Bachofen (Schweiz)] gemischt.
   Die Verpressung zu 500mg Tabletten (11 mm Stempel, rund, flach, mit Facette) erfolgt auf einer instrumentierten Excenter-Tablettiermaschine Korsch EK 0-DMS (Fa. KORSCH, Deutschland) mit dem Auswertesystem Catman 5.0 der Fa. Hottinger Baldwin Messtechnik - HBM (Deutschland).
   Je angewendeter Preßkraft (5, 10, 20 und 30 kN; jeweils +/- 10%) werden mindestens 100 Tabletten zur Auswertung der galenischen Kennzahlen hergestellt.
- Bestimmung der Tablettenhärten, Durchmesser und Höhen: Erweka TBH 30 MD; Fa. Erweka (Deutschland); Durchschnittsdaten aus jeweils 20 Tablettenmessungen pro Preßkraft
- Tablettenabrieb: Friabiliätsprüfgerät Fa.Erweka (Deutschland); Geräteparameter und Ausführung der Messungen gemäß Ph Eur 5. Ausgabe "Friabilität von nicht überzogenen Tabletten"
- Tablettenmasse: Durchschnittswert aus der Wägung von 20 Tabletten; Waage: Mettler AT 201 Fa.Mettler (Deutschland)
- Tablettenzerfall: automatisiertes Tablettenprüfgerät disi 4 der Fa.Biomation (Deutschland); Medium: entsalztes Wasser bei 37°C; Geräteparameter und Ausführung gemäß Ph Eur 5.Ausgabe "Zerfallszeit von Tabletten und Kapseln"
- BET Oberfläche: Auswertung und Durchführung gemäß Literatur "BET Surface Area by Nitrogen Absorption", S. Brunauer et al. (Journal of American Chemical Society, 60, 9,1938) sowie DIN ISO 9277; Geräte: ASAP 2420 Fa.Micromeritics Instrument Corporation (USA); volumetrisches Verfahren; Stickstoff; Einwaage ca. 3g +/- 5% mit Probenvorbereitung (Ausheizung 3,0°C/Min. bis Zieltemperatur 50°C): 10 Stunden/50°C

Die Handhabung, Lagerung, Weiterverarbeitung und Prüfung der Granulate und der Preßlinge erfolgt bei Temperaturen von 19 bis 25 °C und relativen Feuchten im Bereich von 20 bis 35%.

Charakterisierung: Co-Mischung granuliertes 95 Gew.-Teile Mannit mit 5 Gew.-Teilen quervernetzter Na-CMC (95:5) (AcDiSol Typ SD-711; FMC BioPolymer) (2 Beispiele)

| | Erfindungsgemäßes Produkt (1.Beispiel) | Erfindungsgemäßes Produkt (2.Beispiel) |
|---|---|---|
| Schüttdichte | 0,59 g/ml | 0,53 g/ml |
| Fließneigungswinkel | 37,8° | 33,1° |
| Stampfdichte | 0,75 g/ml | 0,66 g/ml |
| Wassergehalt | 0,11 Gew.-% | 0,13 Gew.-% |
| BET-Oberfläche | 1,9 m²/g | 2,6 m²/g |
| Partikelverteilung (Laserbeugung, Vol%) | D(0,10): 16µm | D(0,10): 23µm |
| | D(0,30): 40µm | D(0,30): 72µm |
| | D(0,50): 64µm | D(0,50): 114µm |
| | D(0,75): 112µm | D(0,75): 184µm |
| | D(0,90): 315µm | D(0,90): 269µm |

Tablettierungsdaten: Co-Mischung granuliertes Mannit mit quervernetzter Na-CMC 95:5 (2 Beispiele) im Vergleich zu granuliertem Mannit ohne Zusatz von quervernetzter Na-CMC und im Vergleich zu co-gesprühtem Mannit/quervernetzte Na-CMC 95:5 (Verhältnis jeweils in Gewichtsteilen)

| Produkt | Preßkraft [kN] | Tablettenhärte [N] | Friabilität [Gew.-%] | Zerfall [sec] |
|---|---|---|---|---|
| Erfindungsgemäßes Produkt 1.Beispiel | 5 | 50 | 0,70 | 113 |
| | 10 | 113 | 0,21 | 98 |
| | 20 | 251 | 0,13 | 68 |
| | 30 | 289 | 0,12 | 113 |
| Erfindungsgemäßes Produkt 2.Beispiel | 5 | 63 | 0,22 | 68 |
| | 10 | 142 | 0,16 | 56 |
| | 20 | 287 | 0,12 | 53 |
| | 30 | 310 | 0,13 | 124 |
| 1.Beispiel ohne quervernetzte Na-CMC | 5 | 69 | 0,51 | 102 |
| | 10 | 149 | 0,22 | 315 |
| | 20 | 212 | 0,16 | 257 |
| | 30 | 160 | 100 (Deckeln) | 203 |
| 2.Beispiel ohne quervernetzte Na-CMC | 5 | 91 | 0,19 | 90 |
| | 10 | 169 | 0,17 | 324 |
| | 20 | 200 | 0,20 | 210 |
| | 30 | 179 | 74,81 (Deckeln) | 266 |
| Co-gesprühtes Mannit mit quervernetzter Na-CMC (AcDiSol Typ SD 711)95:5 | 5 | 53 | 0,67 | 48 |
| | 10 | 107 | 0,12 | 199 |
| | 20 | 234 | 0,05 | 434 |
| | 30 | 343 | 0,08 | 479 |

Die erfindungsgemäßen Produkte zeigen im Vergleich mit den gesprühten Mannittypen ohne Zusatz von quervernetzter Na-CMC eine insbesondere im Bereich höherer Preßkräfte verbesserte Tablettenhärte d.h. vor allem keine "Deckelneigung". Zudem sind die Zerfallszeiten der erfindungsgemäßen Produkte deutlich schneller.

Ein co-gesprühtes Produkt, enthaltend 95 Gew.-Teile Mannit und 5 Gew.-Teile quervernetzter Na-CMC liefert, verglichen mit den erfindungsgemäßen Produkten, zwar ähnliche Tablettenhärten (und zeigt auch keine "Deckelneigung") jedoch sind die Zerfallszeiten deutlich erhöht.

**Tablettierungsdaten: Erfindungsgemäße Produkte (2 Beispiele) im Vergleich mit Co-Mischungen aus direkt verpressbaren Mannit-Typen des Marktes (Pearlitol 200 SD, Pearlitol 300, Mannogem 2080, Mannogem 3215) mit quervernetzter Na-CMC (AcDiSol Typ SD-711) 95:5 (Gew.-Teile) und im Vergleich mit einer direkttablettierbaren, für ODT Formulierungen ausgelobten, Co-Sprühung bestehend aus Mannit/Crospovidone/ Polyvinylacetate/Povidone (Ludiflash)**

| Produkt | Preßkraft [kN] | Tablettenhärte [N] | Friabilität [Gew.%] | Zerfall [sec] |
|---|---|---|---|---|
| Erfindungsgemäßes Produkt 1.Beispiel | 5 | 50 | 0,70 | 113 |
| | 10 | 113 | 0,21 | 98 |
| | 20 | 251 | 0,13 | 68 |
| | 30 | 289 | 0,12 | 113 |
| Erfindungsgemäßes Produkt 2.Beispiel | 5 | 63 | 0,22 | 68 |
| | 10 | 142 | 0,16 | 56 |
| | 20 | 287 | 0,12 | 53 |
| | 30 | 310 | 0,13 | 124 |
| Co-Sprühung Mannit/Crospovidone /Poly-Vinylacetat/ Povidone (Ludiflash, BASF) | 5 | 39 | 1,26 | 53 |
| | 10 | 105 | 0,25 | 44 |
| | 20 | 200 | 0,15 | 91 |
| | 30 | 225 | 0,14 | 186 |
| Co-Mischung DC-Mannit (Pearlitol 200 SD) mit quervernetzter Na-CMC 95:5 | 5 | 17 | 4,48 | 62 |
| | 10 | 51 | 0,32 | 62 |
| | 20 | 137 | 0,15 | 56 |
| | 30 | 207 | 0,10 | 83 |
| Co-Mischung DC-Mannit (Pearlitol 300 DC; Roquette) mit quervernetzter Na-CMC 95:5 | 5 | 24 | 3,93 | 149 |
| | 10 | 56 | 0,38 | 138 |
| | 20 | 121 | 0,21 | 115 |
| | 30 | 103 | 75,30 (Deckeln) | 143 |
| Co-Mischung DC-Mannit (Mannogem 2080; SPI) mit quervernetzter Na-CMC 95:5 | 5 | 13 | 42,00 | 104 |
| | 10 | 35 | 2,14 | 96 |
| | 20 | 70 | 45,88 (Deckeln) | 75 84 |
| | 30 | 69 | 80,54 (Deckeln) | |
| Co-Mischung DC-Mannit (Mannogem 3215; SPI) mit quervernetzter Na-CMC 95:5 | 5 | 12 | 95,44 | 69 |
| | 10 | 38 | 1,67 | 66 |
| | 20 | 79 | 0,42 | 61 |
| | 30 | 75 | 85,34 (Deckeln) | 71 |

Im Vergleich mit dem für ODT Formulierungen beworbenen co-gesprühten Mannit/Crospovidone/Polyvinylacetat/Povidone (Ludiflash) lassen sich mit dem erfindungsgemäßen Produkt über einen höheren Tablettenhärtebereich verhältnismäßig schnell zerfallende Tabletten erhalten. Außerdem lassen sich bei vergleichbaren Preßkräften mit dem erfindungsgemäßen Produkt höhere Tablettenhärten erzielen, was für den Galeniker bei der Formulierung schlecht komprimierbarer Wirkstoffe eine Erleichterung bedeutet.

Pearlitol 200 SD, ebenfalls ein gesprühtes Mannit, zeigt im Vergleich zu dem erfindungsgemäßen Produkt ebenfalls ein schlechteres Tablettierverhalten wie auch die anderen direkt verpressbaren Mannit-Typen auf kristalliner bzw. granulierter Basis, die sich durch noch schlechtere Bindungseigenschaften (insbesondere hohe Friabilitäten) auszeichnen.

Die Ergebnisse der vorhergehenden Beispiele und der Vergleichsbeispiele sind in Fig. 1 graphisch dargestellt, wobei die Zerfallszeit der hergestellten Tabletten gegen die erhaltenen Tablettenhärten aufgetragen sind, d.h. Fig 1 zeigt die Abhängigkeit der Tablettenzerfallszeiten von den erzielbaren Tablettenhärten bei den 4 Preßkräften (5, 10, 20 und 30 kN) der erfindungsgemäßen Produkte (2 Beispiele) im Vergleich mit Ludiflash, BASF und einer Mischung Pearlitol 200 SD; Roquette mit quervernetzter Na-CMC (Ac-Di-Sol Typ SD-711, FMC BioPolymers) 95:5. Die erfindungsgemäßen Produkte zeigen im Gegensatz zu den beiden Vergleichen auch bei deutlich höheren Tablettenhärten immer noch schnelle Zerfallszeiten.

Zusammenfassend können mit dem erfindungsgemäßen Produkt ein gegenüber der Stand der Technik bei vergleichbaren Preßkräften härtere Tabletten erhalten werden.

Das Material zeigt somit in einem für die möglichste Schonung der Tablettierwerkzeuge und Tablettiermaschinen bevorzugten Preßkraftbereich bereits sehr gute Bindungseigenschaften. Die sehr gute mechanische Stabilität - gemessen als Friabilität mit deutlich <1% - gewährleistet eine sichere Handhabung der Preßlinge.

Gleichzeitig sind die Zerfallszeiten im Vergleich zu Tabletten, erhalten aus handelsüblichen Produkten des Marktes, über einen weiten Härtebereich hinaus niedrig. Das erfindungsgemäße Material ist daher besonders gut zur Formulierung von pharmazeutischen Zusammensetzungen in Direkttablettierungsprozessen geeignet, und zwar insbesondere auch für die Herstellung von in der Mundhöhle schnell Wirkstoff freisetzender Tabletten. BET-Oberflächen: im folgenden sind die nach der BET-Methode ermittelten Oberflächen der verglichenen Zusammensetzungen zusammengestellt. Wie oben bereits gesagt wurden diese Werte wie in "BET Surface Area by Nitrogen Absorption", S. Brunauer et al. (Journal of American Chemical Society, 60, 9, 1938) bzw. DIN ISO 9277 beschrieben ermittelt. Zur Durchführung der Messungen Geräte der Firma Micromeritics Instrument Corporation (USA) [ASAP 2420] verwendet. Die Messungen wurden nach dem entsprechenden volumetrischen Verfahren mit Stickstoff durch geführt. (Einwaage ca. 3g +/- 5% mit Probenvorbereitung (Ausheizung 3,0°C/Min. bis Zieltemperatur 50°C): 10 Stunden/50°C)

| | |
|---|---|
| Erfindungsgemäßes Produkt 1. Beispiel | 1,9 m²/g |
| Erfindungsgemäßes Produkt 2. Beispiel | 2,6 m²/g |
| Co-gesprühtes mannit mit quervernetztem N-CMC /AcDuSol Typ SD-711) 95:5 | 0,5 m²/g |
| Co-Sprühung Mannit/Crospovidone/Polyvinylacetat/ Povidone (Ludiflash, BASF) | 0,3 m²/g |
| Co-Mischung DC-Mannit (Perlitol 200SD; Roquette) mit quervernetzter Na-CMC 95 : 5 | 0,3 m²/g |
| Co-Mischung DC-Mannit (Perlitol 300DC; Roquette) mit quervernetzter Na-CMC 95 : 5 | 0,6 m²/g |
| Co-Mischung DC-Mannit (Mannogem 2080, SPI) mit quervernetzter Na-CMC 95 : 5 | 0,5 m²/g |
| Co-Mischung DC-Mannit (Mannogem 3215, SPI) mit quervernetzter Na-CMC 95 : 5 | 0,4 m²/g |

Die BET-Daten zeigen, dass sich die erfindungsgemäßen Produkte durch eine deutlich höhere BET-Oberfläche auszeichnen gegenüber anderen Kombinationen aus direkt verpressbaren Mannit-Typen mit Sprengmittel. Diese Vergrößerte Oberfläche ist verbunden mit einer sehr guten Kompressibilität dieser erfindungsgemäßen Materialien, sowie mit schnellen Zerfallseigenschaften der daraus resultierenden, Sprengmittel enthaltenden Tabletten.

## Patentansprüche

1. Co-Mischung zur Herstellung schnell zerfallender Tabletten in einem Direkttablettierungsprozess, bestehend aus 90 - 98 Gew.-Teilen eines gesprühten Mannits und 10 - 2 Gew.-Teilen einer quervernetzten Natriumcarboxymethylcellulose, **dadurch gekennzeichnet, dass** die Co-Mischung eine BET-Oberfläche größer als 1,5 m²/g aufweist bei einem mittleren Partikeldurchmesser (Laser) im Bereich zwischen 60 und 200 µm, zu Tabletten verpressbar ist und nach Kompression bei einer Preßkraft von 20 kN Tabletten mit Härten >250N, einer Friabilität ≤0,14% und einer Zerfallszeit ≤70 Sekunden erhalten werden.

2. Co-Mischung zur Herstellung schnell zerfallender Tabletten in einem Direkttablettierungsprozess gemäß Anspruch 1, bestehend aus 95 Gew.-Teilen einew gesprühten Mannits und 5 Gew.-Teilen einer quervernetzten Natriumcarboxymethylcellulose, **dadurch gekennzeichnet, dass** die Co-Mischung eine BET-Oberfläche größer als 1,5 m²/g aufweist.

3. Co-Mischung gemäß Anspruch 1 oder 2, **dadurch gekennzeichnet, dass** die Co-Mischung eine BET-Oberfläche im Bereich von 1,9 bis 4,0 m²/g aufweist.

4. Co-Mischung gemäß Anspruch 1 oder 2, **dadurch gekennzeichnet, dass** die Co-Mischung eine BET-Oberfläche im Bereich von 1,9 bis 2,6 m²/g aufweist.

5. Co-Mischung nach einem oder mehreren der Ansprüche 1 - 4, charakterisiert durch eine Schüttdichte von 0,45 bis 0,60 g/ml, einer Stampfdichte von 0,60 bis 0,75 g/ml, einem Fließneigungswinkel von 30 bis 38°.

6. Co-Mischung nach einem oder mehreren der Ansprüche 1 - 5, charakterisiert durch einen mittleren Partikeldurchmesser (Laser) im Bereich zwischen 64 und 114 µm.

7. Co-Mischung nach einem oder mehreren der Ansprüche 1 - 6 mit einem Wassergehalt <1 Gew.-%.

8. Wirkstoff- und/oder Aromastoff-haltige Tablettenformulierungen, hergestellt unter Verwendung einer Co-Mischung gemäß einem oder mehreren der Ansprüche 1 - 7 als Trägermaterial.

9. Verwendung eines Trägermaterials gemäß einem oder mehreren der Ansprüche 1 - 7 zur Herstellung von Tablettenformulierungen, welche Vitamine, Mineralstoffe, Spurenelemente, funktionelle Lebensmittelbestandteile enthalten.

10. Verwendung eines Trägermaterials gemäß einem oder mehreren der Ansprüche 1 - 7 zur Herstellung von Tablettenformulierungen, welche Pflanzenbestandteile und Pflanzenextrakte enthalten.

11. Verwendung eines Trägermaterials gemäß einem oder mehreren der Ansprüche 1 - 7 zur Herstellung von Tablettenformulierungen, welche synthetische und natürliche Farbstoffe, natürliche und naturidentische Aromastoffe und andere geschmacksgebende Substanzen wie z.B. Süßstoffe aus der Gruppe Aspartam, Sachcharin, Acesulfam K, Neohesperidin DC, Sucralose; Thaumatin und Steviosid, oder Fruchtaromen, Pfefferminzaromen, Menthol, Fuchtsäuren aus der Gruppe Citronensäure und Weinsäure oder geschmacksgebende Pflanzenauszüge enthalten.

12. Verwendung eines Trägermaterials gemäß einem oder mehreren der Ansprüche 1 - 7 zur Herstellung von Tablettenformulierungen, welche - Substanzen mit pharmakologischer Wirkung wie z.B. Antacida, Antiinfektiva, auch zur lokalen Wirkung im Mund- und Rachenraum, Analgetika einschl. Opioide, Antiallergika, Antiemetika, Antidiarrhoika enthalten.

## Claims

1. Co-mixture for the production of rapidly disintegrating tablets in a direct tableting process, consisting of 90 - 98 parts by weight of a sprayed mannitol and 10 - 2 parts by weight of a crosslinked sodium carboxymethylcellulose, **characterised in that** the co-mixture has a BET surface area of greater than 1.5 m²/g with an average particle diameter (laser) in the range between 60 and 200 µm, can be compressed to give tablets, and, after compression at a compression force of 20 kN, tablets having hardnesses > 250N, a friability ≤ 0.14% and a disintegration time ≤ 70 seconds are obtained.

2. Co-mixture for the production of rapidly disintegrating tablets in a direct tableting process according to Claim 1, consisting of 95 parts by weight of a sprayed mannitol and 5 parts by weight of a crosslinked sodium carboxymethylcellulose, **characterised in that** the co-mixture has a BET surface area of greater than 1.5 m²/g.

3. Co-mixture according to Claim 1 or 2, **characterised in that** the co-mixture has a BET surface area in the range from 1.9 to 4.0 m²/g.

4. Co-mixture according to Claim 1 or 2, **characterised in that** the co-mixture has a BET surface area in the range from 1.9 to 2.6 m²/g.

5. Co-mixture according to one or more of Claims 1 - 4, **characterised by** a bulk density of 0.45 to 0.60 g/ml, a tapped density of 0.60 to 0.75 g/ml, an angle of repose of 30 to 38°.

6. Co-mixture according to one or more of Claims 1 - 5, **characterised by** an average particle diameter (laser) in the range between 64 and 114 µm.

7. Co-mixture according to one or more of Claims 1 - 6 having a water content < 1% by weight.

8. Tablet formulations comprising active compound and/or flavouring, prepared using a co-mixture according to one or more of Claims 1 - 7 as excipient material.

9. Use of an excipient material according to one or more of Claims 1 - 7 for the preparation of tablet formulations which comprise vitamins, mineral substances, trace elements, functional food constituents.

10. Use of an excipient material according to one or more of Claims 1 - 7 for the preparation of tablet formulations which comprise plant constituents and plant extracts.

11. Use of an excipient material according to one or more of Claims 1 - 7 for the preparation of tablet formulations which comprise synthetic and natural dyes, natural and nature-identical flavourings and other flavour-providing substances, such as, for example, sweeteners from the group aspartame, saccharine, acesulfame K, neohesperidin DC, sucralose, thaumatin and stevioside, or fruit flavours, peppermint flavours, menthol, fruit acids from the group citric acid and tartaric acid or flavour-providing plant extracts.

12. Use of an excipient material according to one or more of Claims 1 - 7 for the preparation of tablet formulations which comprise substances having a pharmacological action, such as, for example, antacids, antiinfectives, also for local action in the mouth and throat area, analgesics, including opioids, antiallergics, antiemetics, antidiarrhoeal agents.

## Revendications

1. Co-mélange pour la production de comprimés à désintégration rapide selon un procédé de compression/fabrication directe de comprimés, constitué(e) par 90 à 98 parties en poids d'un mannitol pulvérisé et par 10 à 2 parties en poids d'une carboxyméthylcellulose de sodium réticulée, caractérisé(e) en ce que le co-mélange présente une aire de surface BET supérieure à 1,5 m²/g avec un diamètre de particule moyen (laser) dans la plage entre 60 et 200 µm, peut être comprimé(e) de manière à obtenir des comprimés, et, après compression selon une force de compression de 20 kN, des comprimés qui présentent des duretés > 250 N, une friabilité ≤ 0,14% et un temps de désintégration ≤ 70 secondes sont obtenus.

2. Co-mélange pour la production de comprimés à désintégration rapide selon un procédé de compression/fabrication directe de comprimés selon la revendication 1, constitué(e) par 95 parties en poids d'un mannitol pulvérisé et par 5 parties en poids d'une carboxyméthylcellulose de sodium réticulée, caractérisé(e) en ce que le co-mélange présente une aire de surface BET supérieure à 1,5 m²/g.

3. Co-mélange selon la revendication 1 ou 2, caractérisé(e) en ce que le co-mélange présente une aire de surface BET dans la plage de 1,9 à 4,0 m²/g.

4. Co-mélange selon la revendication 1 ou 2, caractérisé(e) en ce que le co-mélange présente une aire de surface BET dans la plage de 1,9 à 2,6 m²/g.

5. Co-mélange selon une ou plusieurs des revendications 1 à 4, caractérisé(e) par une masse volumique apparente de 0,45 à 0,60 g/ml, une densité après tassement de 0,60 à 0,75 g/ml, un angle d'éboulement de 30 à 38°.

6. Co-mélange selon une ou plusieurs des revendications 1 à 5, caractérisé(e) par un diamètre de particule moyen (laser) dans la plage entre 64 et 114 µm.

7. Co-mélange selon une ou plusieurs des revendications 1 à 6, présentant une teneur en eau < 1 % en poids.

8. Formulations de comprimés comprenant un composé actif et/ou un arôme, préparées en utilisant une co-mélange selon une ou plusieurs des revendications 1 à 7 en tant qu'excipient.

9. Utilisation d'un excipient selon une ou plusieurs des revendications 1 à 7 pour la préparation de formulations de comprimés qui comprennent des vitamines, des substances minérales, des oligo-éléments, des constituants alimentaires fonctionnels.

10. Utilisation d'un excipient selon une ou plusieurs des revendications 1 à 7 pour la préparation de formulations de comprimés qui comprennent des constituants végétaux et des extraits végétaux.

11. Utilisation d'un excipient selon une ou plusieurs des revendications 1 à 7 pour la préparation de formulations de comprimés qui comprennent des colorants synthétiques et naturels, des arômes naturels et des arômes identiques aux arômes naturels et d'autres substances fournissant des arômes, telles que, par exemple, des édulcorants pris parmi le groupe qui est constitué par l'aspartame, la saccharine, l'acésulfame K, la néohespéridine DC, la sucralose, la thaumatine et le stévioside, ou des arômes de fruits, des arômes de menthe poivrée, le menthol, les acides de fruits issus du groupe qui est constitué par l'acide citrique et l'acide tartarique ou des extraits végétaux fournissant des arômes.

12. Utilisation d'un excipient selon une ou plusieurs des revendications 1 à 7 pour la préparation de formulations de comprimés qui comprennent des substances qui présentent une action pharmacologique, telles que, par exemple, les antiacides, les anti-infectieux, également pour une action locale dans la zone buccale et la gorge, les analgésiques, incluant les opioïdes, les antiallergiques, les anti-mimétiques, les agents anti-diarrhéiques.
